Europäisches Patentamt

European Patent Office

Office européen des brevets

(19)

(11) Veröffentlichungsnummer: **0 524 507 B1**

(12) **EUROPÄISCHE PATENTSCHRIFT**

(45) Veröffentlichungstag der Patentschrift: **15.03.95**

(51) Int. Cl.6: **C07C 263/18**, C07C 263/20

(21) Anmeldenummer: **92111822.0**

(22) Anmeldetag: **10.07.92**

(54) **Verfahren zur Reinigung von Polyisocyanaten.**

(30) Priorität: **23.07.91 DE 4124318**

(43) Veröffentlichungstag der Anmeldung:
**27.01.93 Patentblatt 93/04**

(45) Bekanntmachung des Hinweises auf die
Patenterteilung:
**15.03.95 Patentblatt 95/11**

(84) Benannte Vertragsstaaten:
**AT BE CH DE ES FR GB IT LI NL SE**

(56) Entgegenhaltungen:
**DE-B- 1 232 134**
**GB-A- 2 207 671**

**PATENT ABSTRACTS OF JAPAN, vol. 6, no.
165 (C-121)(1043), 28. August 1982; & JP-A-57
082 358**

**CHEMICAL ABSTRACTS, vol. 114, no. 14, 8.
April 1991, Columbus, Ohio, US; abstract no.
124644E, Seite 115, Spalte 1, G. IWAMURA et
al, "Storage-stable one-component coating
compositions"; & JP-A-2 140 277**

(73) Patentinhaber: **BAYER AG**

**D-51368 Leverkusen (DE)**

(72) Erfinder: **Scholl, Hans-Joachim, Dr.**
**Am Feldrain 5**
**W-5000 Köln 80 (DE)**
Erfinder: **Müller, Hanns-Peter, Dr.**
**Hollweg 20**
**W-5068 Odenthal (DE)**
Erfinder: **Welte, Rainer, Dr.**
**Kaiserstrasse 7**
**W-5090 Leverkusen (DE)**

EP 0 524 507 B1

EP 0 524 507 B1

**Beschreibung**

Die Erfindung betrifft ein neues Verfahren zur Reinigung von organischen Polyisocyanaten durch Vermischen der Polyisocyanate mit bestimmten silylierten Verbindungen und gegebenenfalls anschließender Entgasung, oder destillativer Aufarbeitung der so erhaltenen Gemische.

Herstellungsbedingte Verunreinigungen wechselnder Art und Menge in Polyisocyanaten sind Anlaß für Aktivitätsschwankungen, deren Auswirkungen bis in entsprechende Folgeprodukte reichen, so daß eine reproduzierbare und damit wirtschaftliche Verwendung erschwert wird. Insbesondere die bekannten Phosgenierungsprodukte von Anilin-Formaldehyd-Kondensaten (rohe Polyisocyanatgemische der Diphenylmethanreihe) enthalten eine Fülle derartiger Verunreinigungen. Nach Chem. Soc. Rev. 3 (1974) S. 209 ff. handelt es sich hierbei vor allem um Chlor-enthaltende Verunreinigungen, die immer dann Aktivitätsschwankungen verursachen, wenn es sich um "leicht bewegliches", sogenanntes hydrolysierbares Chlor handelt. Eine Verengung der Schwankungsbreite durch Herabsetzung der Menge dieser Verunreinigungen mit der Folge einer Aktivitäts-Normierung und -Verbesserung ist daher sowohl von technischer als auch von wirtschaftlicher Bedeutung.

Nach der Lehre der GB-PS 10 80 717 gelingt die Herabsetzung des Wertes an hydrolysierbarem Chlor (HC-Wert) durch thermische Behandlung bei 180 bis 220°C. Unabhängig vom großen Energieaufwand sind derartige Hochtemperaturverfahren wegen der außerordentlichen Reaktivität von Polyisocyanaten, die unter plötzlicher Wärmeabgabe oligomerisieren können, gefährlich.

Es war daher die der Erfindung zugrundeliegende Aufgabe, ein neues Verfahren zur Reinigung von organischen Polyisocyanaten zur Verfügung zu stellen, welches die angesprochenen Mängel behebt.

Diese Aufgabe konnte mit dem nachstehend näher beschriebenen erfindungsgemäßen Verfahren gelöst werden. Das Prinzip des erfindungsgemäßen Verfahrens besteht darin, den zu behandelnden technischen Polyisocyanaten eine geringe Menge von bestimmten, nachstehend näher beschriebenen silylierten Verbindungen zuzusetzen und die so erhaltenen Gemische gegebenenfalls anschließend zu entgasen oder destillativ aufzuarbeiten.

Gegenstand der Erfindung ist ein Verfahren zur Reinigung von organischen Polyisocyanaten, dadurch gekennzeichnet, daß man organische Polyisocyanate bei 20 bis 150°C mit insgesamt 0,001 bis 1 Gew.-%, bezogen auf die Menge der Polyisocyanate, an Trimethylsilylgruppen aufweisenden Verbindungen der Formeln (Ia) und/oder (Ib), sowie gegebenenfalls (II)

$$(CH_3)_3Si\text{-}NH\text{-}Si(CH_3)_3 \qquad (Ia)$$

$$(CH_3)_3Si\text{-}NH\text{-}CO\text{-}NH\text{-}Si(CH_3)_3 \qquad (Ib)$$

$$X\text{-}[Si(CH_3)_3]_n \qquad (II)$$

vermischt und gegebenenfalls die so erhaltene Mischung nach einer Verweildauer von mindestens 5 Minuten einer Entgasung oder destillativen Aufarbeitung unterzieht, mit der Maßgabe, daß das Molverhältnis der Verbindungen (Ia) und/oder (Ib) zu Verbindungen der Formel (II) bei 1:0 bis 1:1 liegt, und wobei

X für den neutralen Säurerest steht, wie er durch Entfernung der aciden Wasserstoffatome aus einer n-basischen, Sauerstoff enthaltenden Säure mit einem pKa-Wert von maximal 2 erhalten wird, und wobei

n für eine ganze Zahl von 1 bis 3 steht.

Gegenstand der Erfindung sind auch die nach diesem Verfahren gereinigten Polyisocyanate.

Gegenstand der Erfindung ist schließlich auch die Verwendung der so gereinigten Polyisocyanate als Ausgangsmaterial bei der Herstellung von Polyurethankunststoffen nach dem Isocyanat-Polyadditionsverfahren, insbesondere bei der Herstellung von Polyurethanschaumstoffen.

Ausgangsmaterialien für das erfindungsgemäße Verfahren sind beliebige organische Polyisocyanate der aus der Polyurethanchemie an sich bekannten Art.

Geeignete Ausgangspolyisocyanate sind beispielsweise (cyclo)aliphatische Diisocyanate wie beispielsweise 1,6-Diisocyanatohexan (HDI), 1-Isocyanato-3,3,5-trimethyl-5-isocyanatomethyl-cyclohexan (IPDI), 4,4'-Diisocyanato-dicyclohexylmethan (HMDI) und beliebige Gemische derartiger aliphatischer Diisocyanate. Auch aromatische Diisocyanate wie 2,4- und/oder 2,6-Diisocyanatotoluol (TDI) können erfindungsgemäß als Ausgangsmaterial eingesetzt werden. Auch beliebige modifizierte Polyisocyanate können als erfindungsgemäße Ausgangsmaterialien verwendet werden. Von besonderem Interesse sind in diesem Zusammenhang Reaktionsgemische wie sie bei der Trimerisierung eines Teils der Isocyanatgruppen von HDI, IPDI oder Gemischen aus HDI und IPDI zwecks Herstellung der entsprechenden Isocyanuratgruppen aufweisenden

2

Polyisocyanate erhalten werden, und die im wesentlichen aus den genannten Ausgangsdiisocyanaten und den entstehenden Isocyanuratgruppen aufweisenden Polyisocyanaten bestehen.

Besonders bevorzugt kommen jedoch beim erfindungsgemäßen Verfahren gegebenenfalls chemisch modifizierte Polyisocyanate oder Polyisocyanatgemische der Diphenylmethanreihe zum Einsatz.

Hierzu gehören beispielsweise die rohen Phosgenierungsprodukte von Anilin/Formaldehyd-Kondensaten ("Roh-MDI"), aus diesen rohen Gemischen erhaltene Destillationsfraktionen, beispielsweise Polyisocyanate oder Polyisocyanatgemische, die zu 80 bis 100 Gew.-% aus Diisocyanatodiphenylmethan-Isomeren und zu 0 bis 20 Gew.-% aus höher als difunktionellen Polyisocyanaten der Diphenylmethanreihe bestehen, wobei sich die Diisocyanatodiphenylmethan-Isomeren zu 40 bis 100, vorzugsweise 40 bis 80 Gew.-% aus 4,4'-Diisocyanatodiphenylmethan und zum Rest aus 2,4'-Diisocyanatodiphenylmethan und gegebenenfalls 2,2'-Diisocyanatodiphenylmethan zusammensetzen, wobei der Anteil des letztgenannten Diisocyanats bis zu 8 Gew.-%, bezogen auf das Gewicht der Diisocyanate ausmachen kann.

Die genannten Polyisocyanate oder Polyisocyanatgemische können auch in chemisch modifizierter Form zur Anwendung kommen. Unter chemischer Modifizierung sind hierunter insbesondere Urethanisierung, Carbodiimidisierung, Dimerisierung oder Trimerisierung eines Teils der Isocyanatgruppen zu verstehen. Geeignete urethanisierte Ausgangsverbindungen sind insbesondere Umsetzungsprodukte der beispielhaft genannten Polyisocyanate oder Polyisocyanatgemische mit unterschüssigen Mengen an mehrwertigen Alkoholen, insbesondere mit Polypropylenglykolen eines maximalen Molekulargewichts von 700 unter Einhaltung eines NCO/OH-Äquivalentverhältnisses von 10:1 bis 10:3. In Betracht kommende Carbodiimidisierungsprodukte sind insbesondere Carbodiimidgruppen und/oder Uretonimingruppen aufweisende Derivate der beispielhaft genannten Polyisocyanate oder Polyisocyanatgemische, die durch Carbodiimidisierung von 5 bis 30 Gew.-% der Isocyanatgruppen mit Hilfe von Carbodiimidisierungskatalysatoren in an sich bekannter Weise hergestellt worden sind. Geeignete Dimerisierungs- und/oder Trimerisierungsprodukte sind beispielsweise Uretdion und/oder Isocyanuratgruppen aufweisende Derivate der beispielhaft genannten Polyisocyanate oder Polyisocyanatgemische, die durch Dimerisierung und/oder Trimerisierung unter Uretdionund/oder Isocyanuratbildung von 10 bis 30 % der Isocyanatgruppen unter Verwendung von bekannten Trimerisierungskatalysatoren hergestellt worden sind. Beliebige Gemische der chemisch modifizierten Polyisocyanate bzw. Polyisocyanatgemische können selbstverständlich ebenfalls eingesetzt werden.

Ganz besonders bevorzugt werden beim erfindungsgemäßen Verfahren rohe Polyisocyanatgemische der Diphenylmethanreihe mit einer Viskosität bei 24 °C von 10 bis 800, vorzugsweise 15 bis 400 eingesetzt. Diese besonders bevorzugt als Ausgangsmaterialien eingesetzten Polyisocyanatgemische weisen im allgemeinen einen Gehalt an hydrolysierbarem Chlor (HC-Wert) von 0,01 bis 0,2, vorzugsweise 0,02 bis 0,1 Gew.-% auf.

Zur Durchführung des erfindungsgemäßen Verfahrens werden die beispielhaft genannten Ausgangspolyisocyanate mit insgesamt 0,001 bis 1,0, vorzugsweise 0,01 bis 0,3 Gew.-%, bezogen auf Polyisocyanat, an bestimmten Trimethylsilylgruppen aufweisenden Verbindungen versetzt. Die optimale Menge dieser Verbindungen kann leicht durch einen orientierenden Vorversuch ermittelt werden. Die Zugabe erfolgt innerhalb des Temperaturbereichs von 20 bis 150, vorzugsweise 50 bis 120 °C,

Bei den zuzusetzenden Trimethylsilylgruppen aufweisenden Verbindungen handelt es sich um solche der nachstehend genannten Formeln (Ia) und/oder (Ib), sowie gegebenenfalls (II), mit der Maßgabe, daß das Molverhältnis von Verbindungen (Ia) und/oder (Ib) zu (II) bei 1:0 bis 1:1 liegt.

$(CH_3)_3$Si-NH-Si$(CH_3)_3$     (Ia)

$(CH_3)_3$Si-NH-CO-NH-Si$(CH_3)_3$     (Ib)

X-[Si$(CH_3)_3$]$_n$     (II)

In der Formel (II) stehen

X     für den neutralen Säurerest, wie er durch Entfernung der aciden Wasserstoffatome aus einer n-basischen, sauerstoffaufweisenden Säure mit einem pKa-Wert von maximal 2 erhalten wird und

n     für eine ganze Zahl von 1 bis 3.

Als silylierte Sauerstoffsäuren der Formel (II) sind beispielsweise entsprechende silylierte Sulfonsäuren wie Trifluormethansulfonsäuretrimethylsilylester oder Methansulfonsäuretrimethylsilylester, silylierte Ester von Säuren des Phosphors wie Phosphorsäuretris(trimethylsilylester) oder Phosphorsäurediethylestertrimethylsilylester geeignet.

Das Gemisch aus Polyisocyanat und silylierten Verbindungen (Ia) und/oder (Ib), sowie gegebenenfalls (II) wird vorzugsweise nach einem Zeitraum von mindestens 5, vorzugsweise von mindestens 30 Minuten, während welchem das Gemisch bei 20 bis 150, vorzugsweise 50 bis 120°C gehalten wird, einer Entgasung im Vakuum (beispielsweise 100 bis 1 mbar) oder einer destillativen Aufarbeitung unterzogen. Hierunter ist im Falle der Verwendung von destillierbaren Ausgangsisocyanaten deren destillative Reindarstellung, beispielsweise im Dünnschichtverdampfer zu verstehen.

Die erfindungsgemäß behandelten Polyisocyanate weisen vergleichsweise verringerte Aktivitätsschwankungen auf. Dies läßt sich besonders einfach anhand verringerter HC-Werte, gleichbedeutend mit verbesserten Aktivitäten bei vergleichender Verschäumung belegen. Die folgenden Ausführungsbeispiele erläutern die Erfindung. Die angegebenen "HC-Werte" beziehen sich auf den Gehalt an hydrolysierbarem Chlor. Alle Prozentangaben beziehen sich auf das Gewicht.

Beispiele

Ausgangspolyisocyanate

Polyisocyanat 1

Rohes Polyisocyanatgemisch der Diphenylmethanreihe mit einem NCO-Gehalt von 30,9 %, einer Viskosität bei 24°C von 200 mPa.s und einem HC-Wert von 0,088 %.

Polyisocyanat 2

Rohes Polyisocyanatgemisch der Diphenylmethanreihe mit einem NCO-Gehalt von 31,2 %, einer Viskosität bei 24°C von 100 mPa.s und einem HC-Wert von 0,068 %.

Polyisocyanat 3

Polyisocyanatgemisch der Diphenylmethanreihe mit einem NCO-Gehalt von 32,2 %, einer Viskosität bei 24°C von 25 mPa.s und einem HC-Wert von 0,027 %, bestehend aus
59 % 4,4'-Diisocyanatodiphenylmethan
23 % 2,4'-Diisocyanatodiphenylmethan
3 % 2,2'-Diisocyanatodiphenylmethan
15 % höheren Homologen.

Polyisocyanat 4

Polyisocyanatgemisch der Diphenylmethanreihe mit einem NCO-Gehalt von 32,4 %, einer Viskosität bei 24°C von 20 mPa.s und mit einem HC-Wert von 0,082 %, bestehend aus
56 % 4,4'-Diisocyanatodiphenylmethan
29 % 2,4'-Diisocyanatodiphenylmethan
5 % 2,2'-Diisocyanatodiphenylmethan
10 % höheren Homologen.

Beispiel 1 (erfindungsgemäßes Verfahren)

1 kg Polyisocyanat 1 wird unter $N_2$/Rühren auf 90°C erwärmt und mit 1 g Hexamethyldisilazan der Formel (Ia) (HMDS) und 0,1 g Phosphorsäuretris(trimethylsilylester) (PSTMS) versetzt. Man rührt 2 Stunden bei 90°C nach, entgast kurz im Wasserstrahlvakuum und erhält nach Abkühlen ein erfindungsgemäßes Isocyanatgemisch mit einem reduzierten HC-Wert von 0,073 %. NCO-Gehalt und Viskosität bleiben unverändert.

Beispiel 1a (Vergleichsbeispiel)

Beispiel 1 wird ohne Zusatz von HMDS und PSTMS wiederholt. NCO-Gehalt und Viskosität bleiben unverändert, der HC-Wert beträgt 0,087 %.

Die Verschäumung der Isocyanatgemische aus den Beispielen 1 und 1a nach konventioneller Hartschaummethode zeigt eine vergleichsweise verbesserte Aktivität für das erfindungsgemäß gereinigte Polyisocyanatgemisch. Die Liegezeit des Schaumgemisches verringert sich von 39 auf 31 Sekunden.

Entsprechend durchgeführte Beispiele 2 bis 9 sind in Tabelle 2 zusammengestellt:

## Tabelle 1

| | | |
|---|---|---|
| Polyol 1 | 52 Gew.-% | 52 Gew.-% |
| Polyol 2 | 35 " | 35 " |
| Flammschutzmittel | 13 " | 13 " |
| Katalysator | 1,3 " | 1,3 " |
| Stabilisator | 1,2 " | 1,2 " |
| R 11 (CCl$_3$F) | 26 " | 26 " |
| Wasser | 0,5 " | 0,5 " |
| Polyisocyanate [Beispiel 1] | 133 " | - |
| Polyisocyanate [Beispiel 1a] | - | 133 " |
| Mischzeit | 10 sec. | 10 sec. |
| Startzeit | 31 sec. | 39 sec. |

Polyol 1: Polypropylenoxidether, OH value 470,
          Starter: Zucker            81% OH
                   Propylenglykol    14% OH
                   Wasser             5% OH

Polyol 2: Polypropylenoxidether, OH value 450,
          Starter: Zucker            38% OH
                   Ethylenglykol     61% OH
                   Wasser             1% OH

Flame Retardant: Amino⁄phosphonsäureester, OH value
                 450

Katalysator:  N,N'-Dimethylcyclohexylamin

Stabilisator: Polyethersiloxan B 8421 der Firma
              Goldschmidt, Essen.

Tabelle 2

| Beispiele | 2 | 3 | 4 | 5 | 6 | 7 | 8 | 9 |
|---|---|---|---|---|---|---|---|---|
| Ausgangs-produkt | Polyiso-cyanat 1 (1000 g) | Polyiso-cyanat 2 (1000 g) | Polyiso-cyanat 3 (1000 g) | Polyiso-cyanat 4 (1000 g) | Polyiso-cyanat 1 (1000 g) | Polyiso-cyanat 1 (1000 g) | Polyiso-cyanat 4 (1000 g) | Polyiso-cyanat 2 (1000 g) |
| N-Silylver-bindung | HMDS (1 g) | HMDS (1 g) | HMDS (1 g) | HMDS (1 g) | HMDS (1 g) | BSU (1,3 g) | BSU (1,3 g) | BSU (0,6 g) HMDS(0,5 g) |
| silylierte Säure | TRIF (0,2 g) | PSTMS (0,2 g) | PSTMS (0,1 g) | PSTMS (0,1 g) | – | PSTMS (0,1 g) | PSTMS (0,1 g) | PSTMS (0,1 g) |
| Temperatur | 90°C | 90°C | 90°C | 90°C | 90°C | 90°C | 90°C | 90°C |
| Zeit | 2 Std. | 2 Std. | 2 Std. | 2 Std. | 2 Std. | 2 Std. | 2 Std. | 1 1/2 Std. |
| HC-Wert | 0,074 % | 0,058 % | 0,022 % | 0,064 % | 0,077 % | 0,075 % | 0,070 % | 0,057 % |

HMDS: Hexamethyldisilazan

TRIF: Trifluormethansulfonsäuretrimethylsilylester

PSTMS: Phosphorsäuretris(trimethylsilylester)

BSU: N,N'-Bis(trimethylsilyl)harnstoff

**Patentansprüche**

1. Verfahren zur Reinigung von organischen Polyisocyanaten, dadurch gekennzeichnet, daß man organische Polyisocyanate bei 20 bis 150°C mit insgesamt 0,001 bis 1 Gew.-%, bezogen auf die Menge der Polyisocyanate an Trimethylsilylgruppen aufweisenden Verbindungen der Formeln (Ia) und/oder (Ib), sowie gegebenenfalls (II)

$(CH_3)_3$Si-NH-Si$(CH_3)_3$     (Ia)

$(CH_3)_3$Si-NH-CO-NH-Si$(CH_3)_3$     (Ib)

X-[Si$(CH_3)_3$]$_n$     (II)

vermischt und gegebenenfalls die so erhaltene Mischung nach einer Verweildauer von mindestens 5 Minuten einer Entgasung oder destillativen Aufarbeitung unterzieht, mit der Maßgabe, daß das Molverhältnis der Verbindungen (Ia) und/oder (Ib) zu Verbindungen der Formel (II) bei 1:0 bis 1:1 liegt, und wobei

X     für den neutralen Säurerest steht, wie er durch Entfernung der aciden Wasserstoffatome aus einer n-basischen, Sauerstoff enthaltenden Säure mit einem pKa-Wert von maximal 2 erhalten wird, und wobei

n     für eine ganze Zahl von 1 bis 3 steht.

2.  Verfahren gemäß Anspruch 1, dadurch gekennzeichnet, daß man als organische Polyisocyanate chemisch modifizierte Polyisocyanate oder Polyisocyanatgemische der Diphenylmethanreihe verwendet.

3.  Verfahren gemäß Anspruch 1 und 2, dadurch gekennzeichnet, daß man als Verbindungen der Formel (II) Trifluormethansulfonsäuretrimethylsilylester oder Phosphorsäuretris(trimethylsilylester) verwendet.

## Claims

1.  Process for purifying organic polyisocyanates, characterised in that organic polyisocyanates are mixed at 20 to 150°C with a total of from 0.001 to 1% by weight, referred to the quantity of polyisocyanates, of compounds containing trimethylsilyl groups and of the formulae (Ia) and/or (Ib) and optionally (II)

$(CH_3)_3$Si-NH-Si$(CH_3)_3$     (Ia)

$(CH_3)_3$Si-NH-CO-NH-Si$(CH_3)_3$     (Ib)

X-[Si$(CH_3)_3$]$_n$     (II)

and the mixture thus obtained is optionally subjected, after a period of at least 5 minutes, to degassing or to working up by distillation, on condition that the molar ratio of the compounds (Ia) and/or (Ib) to the compounds of the formula (II) is between 1:0 and 1:1, and wherein

X     represents the neutral acid radical which is obtained by removing the acidic hydrogen atoms from an n-basic, oxygen-containing acid having a pKa value of 2 at most, and wherein

n     represents an integer from 1 to 3.

2.  Process according to claim 1, characterised in that the organic polyisocyanates used are chemically modified polyisocyanates or polyisocyanate mixtures of the diphenylmethane series.

3.  Process according to claims 1 and 2, characterised in that the compounds of formula (II) used are trifluoromethane sulphonic acid trimethyl silyl ester or phosphoric acid tris-(trimethyl silyl ester).

## Revendications

1.  Procédé de purification de polyisocyanates organiques, caractérisé en ce que l'on mélange les polyisocyanates organiques à une température de 20 à 150°C avec au total 0,001 à 1 % en masse, par rapport à la quantité de polyisocyanates, de composés contenant des groupes triméthylsilyle ayant les formules (Ia) et/ou (Ib) et éventuellement (II)

$(CH_3)_3$Si-NH-Si$(CH_3)_3$     (Ia)

(CH$_3$)$_3$Si-NH-CO-NH-Si(CH$_3$)$_3$    (Ib)

X-[Si(CH$_3$)$_3$]$_n$    (II)

et on soumet éventuellement le mélange ainsi obtenu, après un temps de contact d'au moins 5 minutes, à un dégazage ou à un traitement par distillation, sous réserve que le rapport en moles des composés (Ia) et/ou (Ib) aux composés de formule (II) soit compris entre 1:0 et 1:1, et
    X représentant le reste neutre d'un acide tel qu'il est obtenu par élimination des atomes d'hydrogène acides d'un acide oxygéné n-basique ayant une valeur de pKa au plus égale à 2, et
    n étant un nombre entier de 1 à 3.

2.  Procédé selon la revendication 1, caractérisé en ce que l'on utilise comme polyisocyanates organiques des polyisocyanates ou des mélanges de polyisocyanates chimiquement modifiés de la série du diphénylméthane.

3.  Procédé selon la revendication 1 et 2, caractérisé en ce que l'on utilise comme composés de formule (II) le trifluorométhanesulfonate de triméthylsilyle ou le phosphate de tris(triméthylsilyle).